# EUROPÄISCHE PATENTSCHRIFT

(11) **EP 3 130 329 B1**
(45) Veröffentlichungstag und Bekanntmachung des Hinweises auf die Patenterteilung: **15.08.2018**
(21) Anmeldenummer: 16173512.1
(22) Anmeldetag: 08.06.2016
(51) Int. Cl.: A61K 8/42, A61K 8/92, A61Q 19/10, A61Q 19/00

(54) **REINIGUNGSPRODUKT AUF ÖLBASIS**
OIL BASED CLEANING PRODUCT
PRODUIT DE NETTOYAGE BASE D'HUILE

(30) Priorität: 12.08.2015 DE 102015215358
(43) Veröffentlichungstag der Anmeldung: 15.02.2017
(73) Patentinhaber: Beiersdorf AG, 20253 Hamburg (DE)
(72) Erfinder: KÖNIG, Sylvia, 21635 Jork (DE); ARGEMBEAUX, Horst, 21465 Wentorf (DE); VIEFHUES, Janie, 22549 Hamburg (DE); KLAWITTER, Helen, 20255 Hamburg (DE)

(56) Entgegenhaltungen:
- EP-A2- 0 557 825
- EP-A2- 1 955 690
- WO-A1-2013/190129
- WO-A2-2012/104025
- DE-A1- 19 843 547
- DE-A1-102004 027 323
- US-A1- 2006 257 353

## Beschreibung

Die vorliegende Erfindung umfasst Reinigungsprodukte auf Ölbasis in halbfester Form.

Die Reinigung der Haut und Hautanhangsgebilde gehört heutzutage in vielen Kulturkreisen zum täglichen Leben, fast vergleichbar wie Essen und Trinken. Bei der Reinigung wird in erster Linie Schmutz, der häufig in Form eines Films auf der Hautoberfläche liegt, von der Hautoberfläche entfernt. Der Schmutzfilm besteht aus festen oder flüssigen Komponenten, die von außen auf die Haut gelangt sind. Ebenfalls zum Schmutzfilm gehören überschüssige Hautlipide und abgestorbene Körperzellen. Durch oberflächenaktive Inhaltsstoffe in den Reinigungszusammensetzungen gelangen die Komponenten des Schmutzfilms in die Waschflotte und werden durch den Abspülprozess von der Haut entfernt. Neben der Beseitigung des Schmutzfilms können jedoch auch, je nach den Reinigungsbedingungen mehr oder weniger, Oberflächenlipide der Haut, beispielsweise Barrierelipide, durch die oberflächenaktiven Substanzen in den Reinigungszubereitungen gelöst und im Abspülvorgang mit entfernt werden. Der Verlust der Hautlipide kann zu Austrocknungserscheinungen und Hautirritationen führen.

Diese unerwünschten Auswirkungen sind besonders bei Menschen mit trockener Haut zu beobachten. Trockene Haut zeichnet sich durch einen Mangel an Feuchtigkeit und Fettstoffen in der obersten Hautschicht aus. Die Haut ist rau, schuppig, glanzlos und wenig elastisch. Es wird häufig ein unangenehmes Spannungsgefühl oder ein Juckreiz empfunden, der insbesondere nach dem Waschen auftreten kann. Dieser Juckreiz führt dazu, dass der Betroffene sich häufig kratzt. Dies wiederum führt dann zu kleinen Verletzungen, die ein Einfallstor für Krankheitserreger sein können. Ebenso können Hautentzündungen auftreten, die sich in Form von Rötungen, Schuppungen, Bläschen und Pusteln ausbilden. Neben den sichtbaren und spürbaren Symptomen lässt sich trockene Haut auch durch Messungen der Rauigkeit, des Wasserverlustes, des Wassergehaltes und des Fettes charakterisieren. Trockene Haut kann erblich bedingt sein und zum Beispiel durch falsche Pflegegewohnheiten, Flüssigkeitsmangel, einseitige Ernährung, Hormonschwankungen und psychische Belastungen verstärkt werden. Aber auch andere Krankheiten können zum Auftreten von trockener Haut führen. Zu nennen sind hier zum einen Hautkrankheiten, wie beispielsweise Neurodermitis, atopische Dermitis, Schuppenflechte und Ichtyosen und zum anderen Stoffwechselerkrankungen wie beispielsweise Schilddrüsenunterfunktion und Diabetes.

Bei der Reinigung trockener Haut sollte beachtet werden, der Haut so wenig wie möglich Fette und Feuchtigkeit zu entziehen. Neben der Beachtung der allgemeinen Regel "so viel wie nötig, so wenig wie möglich", die sich auf die Häufigkeit der Reinigung, insbesondere in Form von Duschbädern oder Wannenbädern bezieht, sollten die Reinigungsprodukte so ausgewählt werden, dass sie besonders mild sind, um die Haut nicht zu reizen und/oder sich durch einen hinreichenden Gehalt an rückfettenden Substanzen auszeichnen.

Besonders geeignet für Menschen mit trockner Haut ist die Anwendung einer Reinigungszubereitung in Form eines Ölbades. Hier können drei Klassen unterschieden werden. Zum einen gibt es Zubereitungen, die im Wesentlichen aus Ölen und Duftstoffen bestehen. Diese Produkte bilden einen Film auf der Wasseroberfläche, der beim Verlassen des Bades auf die Haut aufzieht. In der Regel schäumen derartige Produkt nicht und entfalten auch keine reinigende Wirkung. Der pflegende Aspekt steht bei diesen Produkten im Vordergrund.

Es ist auch möglich, Öl-haltige Badezubereitungen mit Emulgatoren zu versetzen. Bei einer verhältnismäßig geringen Menge an Emulgator tritt eine Trübung bei der Zugabe ins Badewasser auf. Es ist möglich die Emulgatormenge so weit zu erhöhen, dass die Trübung vermieden werden kann.

In einer weiteren Zubereitungsform ölhaltiger Badezubereitungen werden waschaktive Substanzen zugesetzt. Dieser Zusatz führt dazu, dass eine Schaumbildung erfolgen kann. Im Allgemeinen bedeutet der Zusatz dieser waschaktiven Substanzen jedoch, dass die Pflegeleistung abnimmt.

Öl-haltige Badezubereitungen sind an sich bekannt, wie der Stand der Technik zeigt.

Das Dokument DE 29 43 202 beschreibt Mittel mit reinigender und hautpflegender Wirkung auf der Basis von Gemischen aus Tensiden und Ölen, die bevorzugt als sogenannte Pflegeschaumbäder eingesetzt werden, wobei allerdings auch die Verwendung dieser Mittel als Duschzubereitung erwähnt wird. Die beschriebenen Zubereitungen haben einen Gehalt von 20 bis 80 Gew.-% einer wässrigen Tensidlösung, die ihrerseits aus 85 bis 95 Gew.-% Tensid und 5 bis 15 Gew.-% Wasser besteht, sowie einen Ölgehalt von 80 bis 20 Gew.-%. Die waschaktive Komponente dieser Zubereitungen besteht aus Mono-oder Dialkylamin-, Mono-oder Dialkanolamin-oder Alkylalkanolaminsalzen von Fettalkoholschwefelsäureestern.

Die Schrift EP 120 224 beschreibt wirkstoffhaltige Ölbadzubereitungen mit einem Gehalt an 38,75 Gew.-% Sojaöl, 2,00 Gew.-% Rizinusöl, 37,00 Gew.-% Vaselineöl. Als Emulgator werden Polyethylenglycolmono- bzw. -diester offenbart, in Konzentrationen von ca. 10-12 Gew.-%. Der eingesetzte Wirkstoff ist Pelargonsäure.

Das Dokument DD 236014 offenbart ölhaltige kosmetische Mittel, die klar sein müssen. Diese Mittel finden u.a. Verwendung als Badeöle und Cremebäder. Neben 20 bis 80 % Öl, einer wässrigen Aniontensidlösung oder Wasser enthalten diese Zubereitungen eine Kombination aus bestimmten hydrophoben und hydrophilen Emulgatoren bzw. nichtionogenen Tensiden.

In dem Dokument DE 44 24 210 werden kosmetische oder dermatologische Duschzubereitungen mit einem Tensidgehalt von höchstens 55 Gew.-% und einem Ölgehalt von mehr als 45 Gew.-% beschrieben, wobei die Zubereitungen im Wesentlichen wasserfrei sind. Aufgrund des hohen Ölgehalts wirken diese Zubereitungen regenerierend in Bezug auf den allgemeinen Hautzustand. Sie haben dabei gleichzeitig eine gute Schaumentwicklung und eine hohe Reinigungskraft.

Die Schrift EP 0867176 beschreibt Duschzubereitungen, die einen hohen, jedoch im Vergleich zum Stand der Technik deutlich geringeren, Ölgehalt aufweisen. Es wird aber dennoch eine hohe Rückfettung erzielt bei besserer Schaumleistung und Verträglichkeit.

Das Dokument WO 03/051319 offenbart ebenfalls ölhaltige Reinigungsprodukte, in diesem Fall jedoch Reinigungsprodukte auf der Basis von ölhaltigen Mikroemulsionen. Es wird ein Verfahren zur Herstellung dieser ölhaltigen Mikroemulsionen beschrieben. Bei einer konstanten Gesamttensidmenge kann das Primärtensid-/Cotensid-Verhältnis variiert werden.

In dem Dokument WO 2005/065629 wird offenbart, dass ölhaltige, wasserfreie Tensidzubereitungen derart ausgestaltet werden können, dass sie einer Enzymschädigung der Enzyme der oberen Hautschichten entgegenwirken.

Im Dokument WO 2012/104025 werden auch lipidhaltige Badezubereitungen beschrieben, diese werden mit probiotisch aktiven Wirkstoffen versetzt.

Ebenso sind in der Mintel-Datenbank ölhaltige Reinigungszubereitungen zu finden. Beispielhaft seien hier folgende Beispiele erwähnt:
Kneipp Duschöl Schönheitsgeheimnis, Mintel-Nummer 2798965; dieses Duschöl enthält vier natürliche Öle, es wirkt rückfettend und feuchtigkeitsspendend.

Rossmann Wellness&Beauty Duschöl mit Mandelöl und Bambusextrakt, Mintel-Nummer 2303447; dieses Duschöl enthält ebenfalls natürliche Öle und schützt die Haut vor dem Austrocknen.

CD Pflege Duschöl, Mintel-Nummer 1715513; in diesem Duschöl ist Avocadoöl enthalten, das Duschöl reinigt die Haut schonend und schützt vor trockener und empfindlicher Haut. Siehe auch Die DE19843547 A1. Alle diese Zubereitungen haben jedoch Nachteile. Sie sind dünnflüssig, d.h. sie haben in der Regel Viskositätswerte < 1000 mPa·s. Dies führt dazu, dass bei Anwendung derartiger Zubereitungen als Duschzubereitung ein Großteil der Zubereitung leicht von der Hand fließen kann, bevor eine Verteilung auf dem Körper möglich ist. Der Teil der Duschzubereitung, der nicht auf dem Körper verteilt werden konnte, verteilt sich in der Regel auf dem Boden der Duschkabine und kann dazu führen, dass der Boden der Duschkabine rutschig und glitschig wird. Dann besteht durch Ausrutschen eine Verletzungsgefahr.

Zudem kann bei der Anwendung derartiger Produkt beobachtet werden, dass auch die Hände, auf die derartige Produkte aufgebracht wurden, glitschig werden. Bei älteren Badezimmerarmaturen, bei denen ein Nachregeln der Wassertemperatur durch Drehen der entsprechenden Wasserhähne erfolgen muss, führt dies zu Problemen. Das Nachregeln der Temperatur ist erschwert. Wünschenswert sind also ölhaltige Duschzubereitungen, die weniger dünnflüssig sind. Im Stand der Technik werden Zubereitungen offenbart, die diesem Nachteil entgegenwirken, wie DE 10350425. Hier wird ein zähflüssiges Duschöl beschrieben, dem bestimmte Verdicker zugesetzt werden. Diese Verdicker sind Polyamid-Gelbildner, die jedoch dazu führen, dass die Sensorik von Zubereitungen enthaltend diesen Verdicker als negativ empfunden wird. Häufig sind derartige Zubereitungen fadenziehend und klebend.

Die Aufgabe der vorliegenden Erfindung war es also ölhaltige Reinigungszubereitungen zur Verfügung zu stellen, deren Anwendung nicht zu den beschriebenen Nachteilen führt, insbesondere ölhaltige Reinigungszubereitungen zur Verfügung zu stellen, die Viskositätswerte > 5000 mPa·s aufweisen.

Darüber hinaus sollten die Zubereitungen aus möglichst wenigen, weitgehend natürlichen oder Natur-basierten Komponenten bestehen und frei sein von üblicherweise verwendeten polymeren Strukturanten und frei sein von Polyamid-Gelbildnern.

Diese Reinigungszubereitungen sollten eine gute Reinigungsleistung zugleich aber eine sehr gute Pflegeleistung erbringen und für alle Hauttypen geeignet sein, insbesondere jedoch für trockene Haut. Nach der Anwendung der erfindungsgemäßen Produkte sollte es nicht zu Spannungsgefühlen der Haut oder Reizungen der Haut kommen, wie beispielsweise Rötungen oder Juckreiz.

Überraschend wurden diese Aufgaben gelöst durch
ölhaltige Reinigungszubereitungen in halbfester Form, laut Anspruch 1, enthaltend
a. Lipide, wobei die Lipide Gemische von wenigstens einem bei Raumtemperatur in flüssiger Form vorliegenden Lipid und von wenigstens einem bei Raumtemperatur in fester Form vorliegenden Lipid sind und
b. öllösliche Tenside.

Die bei Raumtemperatur flüssigen Lipide werden aus der Gruppe Rizinusöl, Sojaöl und Öl aus Sonnenblumenkernen ausgewählt. In den erfindungsgemäßen Zubereitungen ist wenigstens ein flüssiges Lipid vorhanden. Die festen Lipide werden aus der Gruppe Hydrogenated Vegetable Oil und Hydrogenated Rapeseed Oil ausgewählt. Hydrogenated Vegetable Oil ist beispielsweise erhältlich bei der Firma Dr. Straetmans unter der Handelsbezeichnung Dermofeel Viscolid oder bei der Firma AAK unter der Handelsbezeichnung Akogel Plus. Hydrogenated Rapeseed Oil ist beispielsweise erhältlich bei der Firma ADM unter der Handelsbezeichnung VGB 22. In den erfindungsgemäßen Zubereitungen ist wenigstens ein festes Lipid, ausgewählt aus Hydrogenated Vegetable Oil oder Hydrogenated Rapeseed Oil, vorhanden.

Die bei Raumtemperatur festen und flüssigen Lipide liegen bevorzugt in einem bestimmten Verhältnis zueinander vor, um die gewünschte Konsistenz der Zubereitung zu erzielen. Als vorteilhaft haben sich die Gewichtsverhältnisse der bei Raumtemperatur festen Lipide zu den bei Raumtemperatur flüssigen Lipiden von 60:5 bis 40:1, bevorzugt 55:4 bis 22,5:1 erwiesen.

Die Gemische von wenigstens einem bei Raumtemperatur in flüssiger Form vorliegenden Lipid und von wenigstens einem bei Raumtemperatur in fester Form vorliegenden Lipid liegen einem Gehalt von 45 bis 75 Gew.-%, bevorzugt 50 bis 70 Gew.-%, bezogen auf das Gesamtgewicht der Zubereitung, vor. Mit Hilfe wenigstens eines flüssigen Lipides, in bevorzugten Ausführungsformen mit Rizinusöl oder Sojaöl, wird das Gesamtgewicht der Zubereitung auf 100 Gew.-% gebracht.

Es ist bevorzugt, wenn die Lipide und die öllöslichen Tenside in einem bestimmten Gewichtsverhältnis vorliegen; nämlich in einem Gewichtsverhältnis von Lipiden zu öllöslichen Tensiden von 1: 2 bis 2:1.

Die Reinigungszubereitungen enthalten Tenside, die öllöslich sind. Es hat sich als günstig erwiesen, wenn die Zubereitungen ein Haupttensid enthalten, das bevorzugt wenigstens ein nichtionisches Tensid ist, insbesondere ein Amid einer C₁₀ - C₁₆-Fettsäure, insbesondere bevorzugt ein Monoisopropylamid ist. Haupttenside im Sinne der vorliegenden Erfindung sind diejenigen Tenside, die unter Berücksichtigung aller Tenside in der erfindungsgemäßen Zubereitung, in der überwiegenden Menge vorliegen.

Neben dem Haupttensid können weitere Tenside vorliegen, die als Cotenside bezeichnet werden. Bevorzugt ist es, wenn wenigstens ein Cotensid ein weiteres Amid einer C₁₀ - C₁₆-Fettsäure ist.

Dieses Amid einer C₁₀ - C₁₆-Fettsäure ist verschieden von dem als Haupttensid eingesetzten Amid einer C₁₀ - C₁₆-Fettsäure. Es ist besonders bevorzugt, wenn das Cotensid ein Diethanolamid ist.

Es hat sich ebenfalls als günstig erwiesen, wenn die Cotenside weitere, strukturell andersartige, nichtionische Tenside umfassen. Insbesondere bevorzugt werden als weitere nichtionische Tenside Tenside aus der Gruppe der alkoxylierten Fettalkohole mit einer Kettenlänge von 8 bis 16, bevorzugt 10 bis 14 C-Atomen und einem Alkoxylierungsgrad von 2 bis 10, bevorzugt 2 bis 6 ausgewählt. Der Alkoxylierungsgrad beschreibt die Anzahl der Alkoxygruppen im Molekül. Bevorzugte Alkoxygruppen sind Propylenoxid und Ethylenoxid. Besonders bevorzugt ist Ethylenoxid. Ein Ethylenoxid- Rest kann mit der Bezeichnung EO abgekürzt werden. Als Ethoxilierungsgrad wird die Anzahl der vorhandenen EO-Reste verstanden.

Es ist besonders bevorzugt, wenn die Cotenside Amide einer C₁₀ - C₁₆-Fettsäure und alkoxylierte Fettalkohole mit einer Kettenlänge von 8 bis 16, bevorzugt 10 bis 14 C-Atomen und einem Alkoxylierungsgrad von 2 bis 10, bevorzugt 2 bis 6 umfassen.

Es ist auch möglich, dass die Cotenside noch weitere nichtionische Tenside umfassen.

Der Gehalt an öllöslichen Tensiden beträgt 10 bis 60 Gew.-%, bevorzugt 20 bis 50 Gew.-%, insbesondere bevorzugt 25 bis 40 Gew.-%, bezogen auf das Gesamtgewicht der Zubereitung.

Es ist nicht erforderlich, den erfindungsgemäßen Reinigungszubereitungen polymere Strukturanten zuzusetzen. Unter polymeren Strukturanten werden Polymere verstanden, die sich aus einer Vielzahl chemisch einheitlich aufgebauter Struktureinheiten zusammensetzen und synthetischer Natur sind. Im Sinne der vorliegenden Erfindung werden Komponenten, die sich wiederholende Alkoxy-Einheiten, insbesondere Ethoxy-Einheiten, enthalten, nicht als polymere Strukturanten verstanden. Polymere Strukturanten im Sinne der vorliegenden Erfindung sind insbesondere diejenigen Polymere, die als Struktureinheit (Monomer) wenigstens Acrylsäure und/oder Methacrylsäure und/oder Derivate der Acrylsäure und/oder Methacrylsäure, insbesondere Acrylamid, enthalten. Die erfindungsgemäßen Zubereitungen sind daher frei von polymeren Strukturanten.

Als Polyamidgelbildner werden Substanzen der folgenden chemischen Zusammensetzung verstanden: 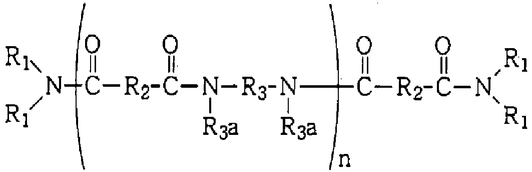 wobei
n = 0-5,
R₁ = Kohlenwasserstoff mit 1-22 Kohlenstoffatomen,
R₂ = Kohlenwasserstoff mit 2-42 Kohlenstoffatomen,
R₃ = Kohlenwasserstoff mit 2-36 Kohlenstoffatomen, enthaltend 0-3 Heteroatome, gewählt aus der Gruppe Sauerstoff und Stickstoff,
R₃^{a} = H, C1 -C10 -Kohlenstoffrest bedeuten. Die erfindungsgemäßen Reinigungszubereitungen sind frei von Polyamidgelbildnern.

In einer vorteilhaften Ausführungsform enthalten die erfindungsgemäßen Reinigungszubereitungen keine Polyole.

In einer anderen Ausführungsform, die ebenfalls vorteilhaft ist, sind Polyole enthalten, jedoch nicht mehr als 10 Gew.-%, bezogen auf das Gesamtgewicht der Zubereitung.

Die erfindungsgemäßen Reinigungszubereitungen zeichnen sich dadurch aus, dass der Zusatz von Konservierungsmitteln nicht erforderlich ist. Daher sind die erfindungsgemäßen Reinigungszubereitungen frei von Konservierungsmitteln.

Konservierungsmittel sind diejenigen konservierenden Substanzen, die gemäß Kosmetikverordnung für Deutschland und gemäß der Verordnung (EG) Nr. 1223/2009 über kosmetische Mittel für den Einsatz in kosmetischen Produkten für Europa zugelassen sind.

Um die Wirkung von Konservierungsstoffen zu ergänzen oder zu verstärken, werden kosmetischen Zubereitungen Substanzen zugesetzt, die die Wirkung von Konservierungsmitten verstärken, so dass es möglich ist, die Konzentration des Konservierungsmittels abzusenken. Diese Substanzen werden im Folgenden als verstärkende Substanzen bezeichnet.

Verstärkende Substanzen sind beispielsweise folgende Verbindungen: Alcohol Denat., Propylene Glycol, Ethylhexylglycerin, 1,2-Hexanediol, Trisodium EDTA, Methylpropanediol, Butylene Glycol, Polyaminopropyl Biguanide, und Caprylyl Glycol. Ebenso können ausgewählte kationische Polymere wie Polyquaternium- und Polyquaternium-10 oder ausgewählte kationische Guar-Derivate wie beispielsweise Guar Hydroxpropyl Trimethylammonium Chloride und ausgewählte kationische Tenside wie beispielsweise Cetyl Trimethylammonium Chloride als verstärkende Substanzen wirken.

Die erfindungsgemäßen Zubereitungen sind frei von den verstärkenden Substanzen.

Gleichwohl ist es denkbar, in alternativen Ausführungsformen Konservierungsmittel und/oder verstärkende Substanzen einzusetzen.

Den erfindungsgemäßen Reinigungszubereitungen werden keine Farbstoffe zugesetzt. Die erfindungsgemäßen Zubereitungen sind also frei von Farbstoffen. Gleichwohl ist es denkbar, in alternativen Ausführungsformen Farbstoffe einzusetzen.

Um die Stabilität der erfindungsgemäßen Zubereitungen zu gewährleisten, müssen geringe Mengen an Wasser zugesetzt werden. Daher enthalten die erfindungsgemäßen Zubereitungen 0,02 bis 0,09 Gew.-%, bevorzugt 0,04 bis 0,08 Gew.-% Wasser, bezogen auf die Gesamtzubereitung.

Weiterhin förderlich für die Stabilität der erfindungsgemäßen Reinigungszubereitungen ist der Zusatz von ausgewählten organischen Säuren wie Milchsäure oder Zitronensäure, bevorzugt Zitronensäure. Zitronensäure liegt in den erfindungsgemäßen Zubereitungen in einer Konzentration von 0,05 bis 0,2 Gew.-%, bevorzugt mit 0,075 bis 0,15 Gew.-% vor, bezogen auf das Gesamtgewicht der Zubereitung.

Die erfindungsgemäßen Reinigungsprodukte sind überaus vorteilhafte Zubereitungen, die ohne den Einsatz von polymeren Strukturanten, Polyamidgelbildnern, Konservierungsmitteln und Farbstoffen auskommen und darüber hinaus aus wenigen, weitgehend natürlichen oder Natur-basierten Rohstoffen zusammengesetzt sind. Daher sind die erfindungsgemäßen Reinigungszubereitungen überaus geeignet zur milden und pflegenden Reinigung bei trockener und/oder empfindlicher Haut und/oder Altershaut, insbesondere jedoch bei trockener Haut.

Ein weiterer Gegenstand der vorliegenden Erfindung ist daher die Verwendung von erfindungsgemäßen Reinigungszubereitungen zur milden und pflegenden Reinigung bei trockener und/oder empfindlicher Haut und/oder Altershaut.

Die erfindungsgemäßen Reinigungszubereitungen sind vorteilhaft zu verwenden als Reinigungszubereitungen. Ebenso vorteilhaft ist die Verwendung als Gesichtsreinigungsprodukt. Denkbar sind auch andere Anwendungsformen, beispielsweise als Zubereitung zur Unterstützung der Körperrasur.

Folgende *Tenside* können in den Zubereitungen vorteilhaft verwendet werden:
Die günstig zu verwendenden Amide der Fettalkoholsulfate bzw. der Fettalkoholethersulfate weisen folgende Struktur auf:

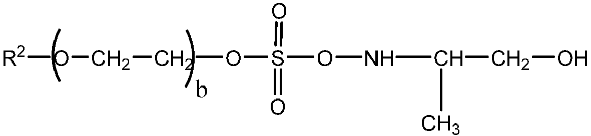

Dabei kann b Werte von 0 bis 10, vorteilhaft 1 bis 5 annehmen. R² wird gewählt aus der Gruppe der verzweigten und unverzweigten Alkylgruppen mit 6 bis 24 Kohlenstoffatomen. Zwingend verwendetes Fettalkoholethersulfat ist MIPA-Laurethsulfat. Vorteilhaft weisen die günstig zu verwendenden Fettalkoholethoxylate folgende Struktur auf:

R³-(O-CH₂-CH₂-)_{c}-OH

Dabei kann c Werte von 2 bis 10 annehmen, bevorzugt von 2 bis 6. R³ wird gewählt aus der Gruppe der verzweigten und unverzweigten Alkylgruppen mit 8 bis 16 Kohlenstoffatomen, insbesondere 10 bis 14 Kohlenstoffatome. Zwingend verwendetes Fettalkoholethoxylat ist Laureth-4.

Vorteilhaft weisen die günstig zu verwendenden Fettsäuremono- bzw. - diethanolamide folgende Strukturen auf: 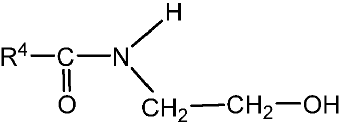 bzw.

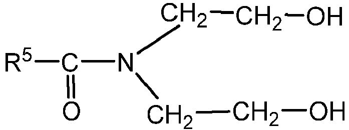

R⁴ bzw. R⁵ werden dabei gewählt aus der Gruppe der verzweigten und unverzweigten Alkylgruppen und/oder Alkenylgruppen mit 6 bis 24 Kohlenstoffatomen, insbesondere mit 10 bis 16 Kohlenstoffatomen. Zwingend verwendetes Fettsäurediethanolamid ist Kokosfettsäurediethanolamid (Cocamide DEA). Natürliche Kokosfettsäure enthält als wesentliche Bestandteile Laurinsäure zu 44-51 Gew.-%, Myristinsäure zu 13-18 Gew.-%, Palmitinsäure zu 8-10 Gew.-%, Caprylsäure zu 6-9 Gew.-%, Caprinsäure zu 6-10 Gew.-%, Ölsäure zu 5- 8 Gew.-%, Stearinsäure zu 1-3 Gew.-%, Linolsäure zu 0-2 Gew.-% und Capronsäure zu 0-1 Gew.-%. Erfindungsgemäss ist es alleine Gemische aus MIPA-Laurethsulfat, Laureth-4 und Kokosfettsäurediethanolamid einzusetzen. Solche Gemische sind beispielsweise unter der Bezeichnung ZETESOL® 100 von der Firma Zschimmer & Schwarz Chemische Fabriken, Lahnstein/Rhein, oder TEXAPON® WW 99 von der Henkel KGaA, Düsseldorf, erhältlich. *Öle* werden vorzugsweise gewählt aus der Gruppe der Triglyceride folgender Struktur: 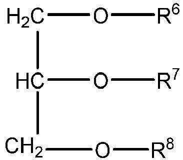 wobei R⁶, R⁷ und R⁸ unabhängig voneinander gewählt werden aus der Gruppe der verzweigten und unverzweigten, Alkylcarboxyl bzw. Alkenylcarboxylgruppen mit 12 bis 24 Kohlenstoffatomen. Es ist gegebenenfalls vorteilhaft, wenn eine oder mehrere aliphatische Wasserstoffatome der Alkylcarboxyl- bzw. Alkenylcarboxylgruppen durch Hydroxylgruppen substituiert sind.

Für die Auswahl der bei Raumtemperatur flüssigen Lipide ist es insbesondere vorteilhaft, wenn R⁶, R⁷ und/oder R⁸ 16 bis 20 Kohlenstoffatome aufweisen und aus der Gruppe der einfach bis dreifach ungesättigten Carbonsäurereste gewählt werden.

Wenn R⁶, R⁷ und/oder R⁸ Hydroxylgruppen tragen, ist der bevorzugte Alkenylcarboxylrest der Ricinolsäurerest.

Besonders vorteilhaft ist, die erfindungsgemäßen bei Raumtemperatur flüssigen Lipide aus der Gruppe Sojaöl, Rizinusöl und Sonnenblumenkernöl auszuwählen.

Für die Auswahl der bei Raumtemperatur festen Lipide ist es vorteilhaft, wenn diese einen Schmelzpunkt oder Schmelzbereich in einem Fenster von 50 bis 70°C, eine Jodzahl zwischen 0 und 5 und einen Gehalt an freien Fettsäuren von 0 bis 0,8 Gew.-% haben.

*Polyole* in Sinne der vorliegenden Erfindung sind mehrwertige Alkohole. Es handelt sich um niedermolekulare Verbindungen, die mindestens zwei Hydroxylgruppen tragen. Erfindungsgemäß bevorzugt sind die Verbindungen Propylenglycol und Glycerin, insbesondere bevorzugt ist Glycerin.

Die Viskosität ausgewählter Zubereitungen wurde mit einem deformationsgesteuerten Rotationsrheometer ARES der Firma TA Instruments durchgeführt. Die Messung erfolgte mittels Rate Sweep Test, bei dem die Scherrate stufenförmig von 0,1 bis 1000 pro Sekunde mit 5 Messpunkten pro Dekade erhöht wird. Hierbei wird vor jedem Messpunkt während einer Wartezeit von 15 Sekunden die jeweilige Scherrate gehalten und in weiteren 5 Sekunden Messdaten erfasst aus denen dann die Software den Messpunkt ermittelt. Als Geometrie kam ein Kegel-Platte-Messsystem zum Einsatz mit einem Durchmesser von 25 mm und einem Kegelwinkel von 0,02 rad. Die Temperatur wurde mittels eines Peltiertemperiersystems an der unteren Messplatte auf 25°C gehalten. Beim Befüllen wurde der Rand 0,1 mm vor dem Erreichen des Messspalts bereinigt. Direkt nach dem Befüllen wurde vor dem Test eine Wartezeit von 2 Minuten zur Temperierung eingehalten. Die Viskosität wurde bei einer Scherrate von 10 pro Sekunde ausgewertet.

Um die Wirksamkeit der erfindungsgemäßen Zubereitungen zu belegen, wurde eine ausgewählte Zubereitung, TARTINE:216, in einem In-Use-Screening eingesetzt. Die Zubereitung TARTINE:216 wurde an 100 Probandinnen verteilt, die das Produkt mindestens viermal innerhalb von 12 Tagen benutzen mussten. Es wurde ein Fragebogen ausgeteilt, mit dem verschiedene Produktleistungen abgefragt wurden. Von 100 Fragebögen wurden 80 zurückgegeben und konnten ausgewertet werden. Die Probandinnen waren alle weiblich, im Schnitt 52 Jahre alt. Die Fragen konnten auf einer Skala von 1 (stimme nicht zu) bis 7 (stimme zu) beantwortet werden. Die Antworten wurden in Bezug auf die Ermittlung des jeweiligen Mittelwertes, der absoluten und relativen Häufigkeit ausgewertet. Zusätzlich wurden sogenannte Topboxes und Lowboxes ermittelt, d.h. die Antworten in Bezug auf jeweils besten und zweitbesten bzw. die schwächsten und zweit schwächsten Aussagen gruppiert.

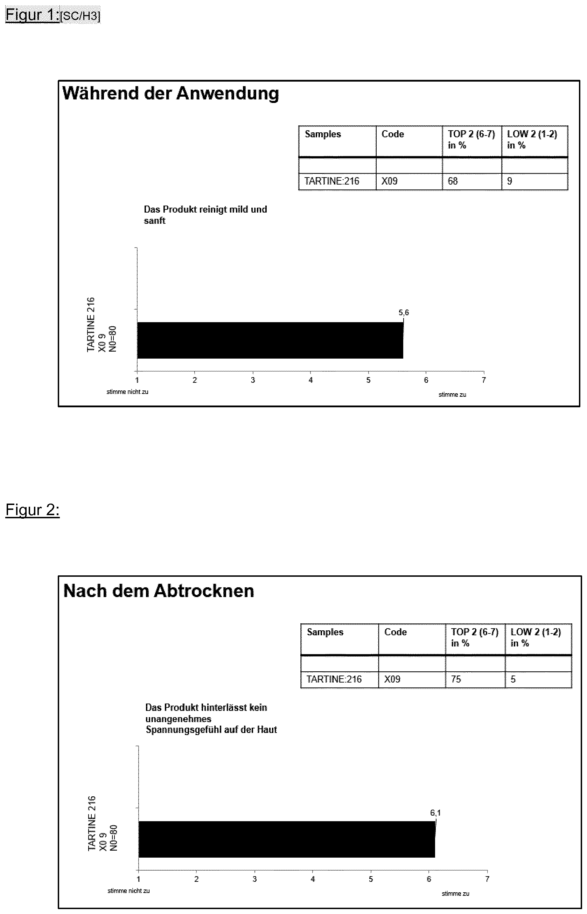

Die Reinigung durch die Zubereitung TARTINE:216 wurde von 68 % der Anwender während der Anwendung mit mild und sanft beurteilt. Der Prozentwert setzt sich aus den Werten der höchsten und zweithöchsten Zustimmung zusammen (siehe Figur 1).

Ebenso wurde abgefragt, ob die Zubereitung TARTINE:216 nach dem Abtrocknen ein Spannungsgefühl auf der Haut hinterlässt. Dies verneinten 75 % der Anwender. Der Prozentwert setzt sich aus den Werten der höchsten und zweithöchsten Zustimmung zusammen (siehe Figur 2).

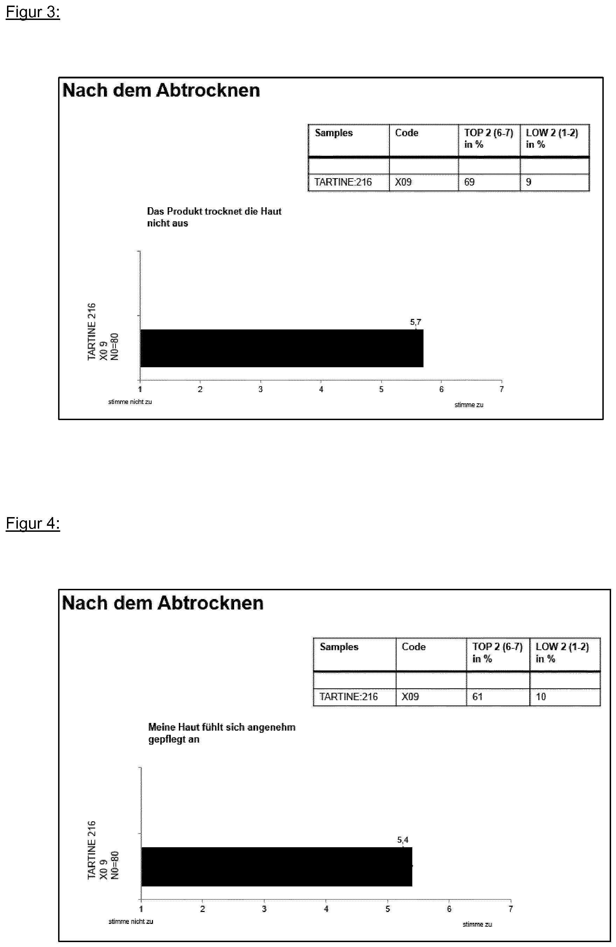

Weiterhin sollten die Anwender nach dem Abtrocknen beurteilen, ob die Zubereitung TARTINE:216 die Haut austrocknet. Dies verneinten 69 % der Anwender. Der Prozentwert setzt sich aus den Werten der höchsten und zweithöchsten Zustimmung zusammen (siehe Figur 3).

Nach dem Abtrocknen fühlt sich die Haut nach Anwendung der Zubereitung TARTINE:216 angenehm gepflegt an. Dies bejahten 61 % der Anwender. Der Prozentwert setzt sich aus den Werten der höchsten und zweithöchsten Zustimmung zusammen (siehe Figur 4).

Das in dem IN-USE-Screening verwendete Produkt, TARTINE:216 hat folgende Zusammensetzung:

| **Handelsname, Hersteller** | **TARTINE 216** |
|---|---|
| Fragrance | 2,00 |
| AKOGEL PLUS, AAK | 50,00 |
| VGB 22, ADM | 3,00 |
| Sojaöl, Gustav Heess | 3,77 |
| CitronensäureMonohydrat , Jungbunzlauer | 0,12 |
| WASSER VES | 0,06 |
| Jonol CP, Technochemie | 0,05 |
| Zetesol 100, Zschimmer&Schwarz | 41,00 |

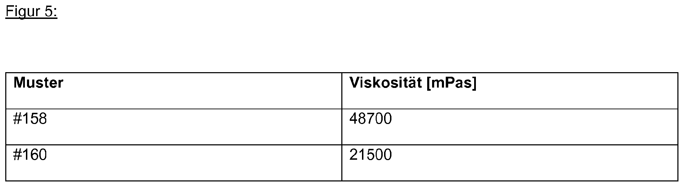

In der Figur 5 sind die Messergebnisse von Viskositätsmessungen dargestellt. Die Messung erfolgte unter den oben beschriebenen Bedingungen. Vermessen wurden die Proben , #158, entsprechend der Zusammensetzung TARTINE 216 und #160.

| **Handelsname, Hersteller** | **# 158 (TARTINE 216)** | **# 160 (TARTINE 273)** |
|---|---|---|
| Fragrance | 2,00 | 2,00 |
| AKOGEL PLUS, AAK | 50,00 | 47,00 |
| VGB 22, ADM | 3,00 | 2,82 |
| Sojaöl, Gustav Heess | 3,77 | 6,95 |
| CitronensäureMonohydrat, Jungbunzlauer | 0,12 | 0,12 |
| WASSER VES | 0,06 | 0,06 |
| Jonol CP, Technochemie | 0,05 | 0,05 |
| Zetesol 100, Zschimmer&Schwarz | 41,00 | 41,00 |

### Beispiele:

| **Handelsname, Hersteller** | **INCI** | **1** | **2** | **3** | **4** | **5** |
|---|---|---|---|---|---|---|
| Fragrance | Parfum | 1,80 | 2,00 | 2,00 | 2,20 | 1,90 |
| AKOGEL PLUS, AAK | Hydrogenated Vegetable Oil | 45,00 | 50,00 | 42,00 | 55,00 | 57,00 |
| Dermofeel Viscolid, Dr. Straetmans | Hydrogenated Vegetable Oil | 3,50 | 0,00 | 0,00 | 0,00 | 1,00 |
| VGB 22, ADM | Hydrogenated Rapeseed Oil | 0,00 | 2,40 | 4,40 | 0,00 | 1,00 |
| Sojaöl*, Gustav Heess | Glycine Soja Oil + Ricinus Communis Seed Oil + Propyl Gallate | 0,00 | 3,60 | 4,20 | ad.q. | ad.q. |
| Castor oil virgin, Gustav Heess | Ricinus Communis Seed Oil | ad.q. | 1,00 | ad.q. | 0,00 | 0,00 |
| Sunflower Oil refined solvent, Gustav Heess | Helianthus Annuus Seed Oil | 0,00 | ad q | 0,00 | 0,00 | 2,40 |
| CitronensäureMonohydrat, Jungbunzlauer | Citric Acid | 0,11 | 0,10 | 0,11 | 0,10 | 0,13 |
| WASSER VES | Aqua | 0,06 | 0,05 | 0,06 | 0,05 | 0,07 |
| Jonol CP, Technochemie | BHT | 0,00 | 0,07 | 0,06 | 0,05 | 0,04 |
| Zetesol 100, Zschimmer&Schwarz | MIPA Laureth Sulfate + Laureth-4 + Cocamide DEA | 46,00 | 39,00 | 41,00 | 42,00 | 34,00 |

| | | | | | | |
|---|---|---|---|---|---|---|
| *99,919% Glycine Soja (Soybean) Oil 0,07% Ricinus Communis (Castor) Seed Oil 0,01% Propyl Gallate 0,001% Citric Acid | | | | | | |

## Patentansprüche

1. Ölhaltige Reinigungszubereitungen in halbfester Form enthaltend
a) Lipide, wobei die Lipide Gemische von wenigstens einem bei Raumtemperatur in flüssiger Form vorliegenden Lipid und von wenigstens einem bei Raumtemperatur in fester Form vorliegenden Lipid sind und
wobei das Gewichtsverhältnis des wenigstens einen bei Raumtemperatur festen Lipids zu dem wenigstens einen bei Raumtemperatur flüssigen Lipids 55:4 bis 22,5:1, beträgt und
wobei die Lipide mit einem Gehalt von 50 bis 70 Gew.-%, bezogen auf das Gesamtgewicht der Zubereitung, vorliegen und
wobei die bei Raumtemperatur festen Lipide ausgewählt werden aus der Gruppe Hydrogenated Vegetable Oil und Hydrogenated Rapeseed Oil und
wobei die bei Raumtemperatur flüssigen Lipide ausgewählt werden aus der Gruppe Rizinusöl, Sojaöl und Öl aus Sonnenblumenkernen,
b) Gemische aus MIPA-Laurethsulfat, Laureth-4 und Kokosfettsäurediethanolamid als öllösliche Tenside.

2. Reinigungszubereitungen nach Anspruch 1 **dadurch gekennzeichnet, dass** der Gehalt an öllöslichen Tensiden 10 bis 60 Gew.-%, bevorzugt 20 bis 50 Gew.-%, insbesondere bevorzugt 25 bis 40 Gew.-% beträgt, bezogen auf das Gesamtgewicht der Zubereitung.

3. Reinigungszubereitungen nach Anspruch 1 und/oder Anspruch 2 **dadurch gekennzeichnet, dass** die Reinigungszubereitung frei ist von polymeren Strukturanten und/oder Polyamidgelbildnern,
wobei die polymeren Strukturanten Polymere sind, die als Struktureinheit (Monomer) wenigstens Acrylsäure und/oder Methacrylsäure und/oder Derivate der Acrylsäure und/oder Methacrylsäure, insbesondere Acrylamid, enthalten.

4. Reinigungszubereitungen nach wenigstens einem der vorstehenden Ansprüche **dadurch gekennzeichnet, dass** die Reinigungszubereitung keine Polyole enthält oder nicht mehr als 10 Gew.-%, bezogen auf das Gesamtgewicht der Zubereitung, an Polyolen enthält.

5. Reinigungszubereitungen nach wenigstens einem der vorstehenden Ansprüche **dadurch gekennzeichnet, dass** die Reinigungszubereitung frei ist von Konservierungsmitteln und/oder verstärkenden Substanzen.

6. Reinigungszubereitungen nach wenigstens einem der vorstehenden Ansprüche **dadurch gekennzeichnet, dass** die Reinigungszubereitung frei ist von Parfümrohstoffen.

7. Reinigungszubereitungen nach wenigstens einem der vorstehenden Ansprüche **dadurch gekennzeichnet, dass** die Reinigungszubereitung frei ist von Farbstoffen.

## Claims

1. Oil-containing cleansing preparations in semisolid form, comprising
a) lipids, the lipids being mixtures of at least one lipid present in liquid form at room temperature and of at least one lipid present in solid form at room temperature, and
the weight ratio of the at least one lipid solid at room temperature to the at least one lipid liquid at room temperature being 55:4 to 22.5:1, and
the lipids being present with a content of 50 to 70 wt%, based on the total weight of the preparation,
the lipids solid at room temperature being selected from the group of hydrogenated vegetable oil and hydrogenated rapeseed oil, and
the lipids liquid at room temperature being selected from the group of castor oil, soyabean oil and oil from sunflower seeds,
b) mixtures of MIPA laureth sulfate, laureth-4 and coconut-fatty acid diethanolamide as oil-soluble surfactants.

2. Cleansing preparations according to Claim 1, **characterized in that** the content of oil-soluble surfactants is 10 to 60 wt%, preferably 20 to 50 wt%, especially preferably 25 to 40 wt%, based on the total weight of the preparation.

3. Cleansing preparations according to Claim 1 and/or Claim 2, **characterized in that** the cleansing preparation is free from polymeric structurants and/or polyamide gel formers, the polymeric structurants being polymers which comprise as structural unit (monomer) at least acrylic acid and/or methacrylic acid and/or derivatives of acrylic acid and/or methacrylic acid, especially acrylamide.

4. Cleansing preparations according to at least one of the preceding claims, **characterized in that** the cleansing preparation contains no polyols or contains not more than 10 wt% of polyols, based on the total weight of the preparation.

5. Cleansing preparations according to at least one of the preceding claims, **characterized in that** the cleansing preparation is free from preservatives and/or reinforcing substances.

6. Cleansing preparations according to at least one of the preceding claims, **characterized in that** the cleansing preparation is free from perfume raw materials.

7. Cleansing preparations according to at least one of the preceding claims, **characterized in that** the cleansing preparation is free from dyes.

## Revendications

1. Préparations détergentes contenant de l'huile sous forme semi-solide, contenant :
a) des lipides, les lipides étant des mélanges d'au moins un lipide présent sous forme liquide à température ambiante et d'au moins un lipide présent sous forme solide à température ambiante, et
le rapport en poids entre ledit au moins lipide solide à température ambiante et ledit au moins un lipide liquide à température ambiante étant de 55:4 à 22,5:1, et
les lipides étant présents en une teneur de 50 à 70 % en poids, par rapport au poids total de la préparation, et
les lipides solides à température ambiante étant choisis dans le groupe constitué par l'huile végétale hydrogénée et l'huile de colza hydrogénée, et
les lipides liquides à température ambiante étant choisis dans le groupe constitué par l'huile de ricin, l'huile de soja et l'huile de graines de tournesol,
b) des mélanges de laureth-sulfate de MIPA, de laureth-4 et de diéthanolamine d'acide gras de coco en tant que tensioactifs solubles dans les huiles.

2. Préparations détergentes selon la revendication 1, **caractérisées en ce que** la teneur en tensioactifs solubles dans les huiles est de 10 à 60 % en poids, de préférence de 20 à 50 % en poids, de manière particulièrement préférée de 25 à 40 % en poids, par rapport au poids total de la préparation.

3. Préparations détergentes selon la revendication 1 et/ou la revendication 2, **caractérisées en ce que** la préparation détergente est exempte de structurants polymères et/ou d'agents gélifiants polyamide,
les structurants polymères étant des polymères qui contiennent en tant qu'unité structurale (monomère) au moins de l'acide acrylique et/ou de l'acide méthacrylique et/ou des dérivés de l'acide acrylique et/ou de l'acide méthacrylique, notamment l'acrylamide.

4. Préparations détergentes selon au moins l'une quelconque des revendications précédentes, **caractérisées en ce que** la préparation détergente ne contient pas de polyols ou ne contient pas plus de 10 % en poids, par rapport au poids total de la préparation, de polyols.

5. Préparations détergentes selon au moins l'une quelconque des revendications précédentes, **caractérisées en ce que** la préparation détergente est exempte de conservateurs et/ou de substances renforçantes.

6. Préparations détergentes selon au moins l'une quelconque des revendications précédentes, **caractérisées en ce que** la préparation détergente est exempte de matières premières parfumées.

7. Préparations détergentes selon au moins l'une quelconque des revendications précédentes, **caractérisées en ce que** la préparation détergente est exempte de colorants.
